# EUROPÄISCHE PATENTSCHRIFT

(11) **EP 1 963 516 B1**
(45) Veröffentlichungstag und Bekanntmachung des Hinweises auf die Patenterteilung: **23.02.2011**
(21) Anmeldenummer: 06819032.1
(22) Anmeldetag: 14.12.2006
(51) Int. Cl.: C12P 7/62, C12P 41/00, C12P 13/00

(54) **VERFAHREN ZUR ENANTIOSELEKTIVEN ENZYMATISCHEN REDUKTION VON HYDROXYKETOVERBINDUNGEN**
METHOD FOR THE ENANTIOSELECTIVE ENZYMATIC REDUCTION OF HYDROXYKETO COMPOUNDS
PROCEDE DE REDUCTION ENZYMATIQUE ENANTIOSELECTIVE DE COMPOSES HYDROXYCETO

(30) Priorität: 19.12.2005 AT 20272005
(43) Veröffentlichungstag der Anmeldung: 03.09.2008
(73) Patentinhaber: IEP GmbH, 65203 Wiesbaden (DE)
(72) Erfinder: GUPTA, Antje, 65207 Wiesbaden (DE); BOBKOVA, Maria, 65207 Wiesbaden (DE); TSCHENTSCHER, Anke, 65347 Eltville-Hattenheim (DE)
(74) Vertreter: Schwarz, Albin
(86) Internationale Anmeldenummer: PCT/EP2006/012037
(87) Internationale Veröffentlichungsnummer: WO 2007/073875

(56) Entgegenhaltungen:
- EP-A1- 1 152 054
- WO-A-03/078615
- WO-A-2004/111083
- WO-A-2005/108593
- US-A- 6 001 615
- DATABASE UniProt [Online] 11. Juli 2006 (2006-07-11), COPELAND ET AL: "Short-chain dehydrogenase/reductase from Rubrobacter xylanophilus DSM 9941" XP002429149 gefunden im EBI accession no. Q1AU84_RUBXD Database accession no. Q1AU84
- DATABASE UniProt [Online] 1. Februar 2005 (2005-02-01), TAKAMI ET AL: "Short-chain dehydrogenase from Geobacillus kaustophilus" XP002429150 gefunden im EBI accession no. Q5KZB8_GEOKA Database accession no. Q5KZB8
- DATABASE UniProt [Online] 22. November 2005 (2005-11-22), COPELAND ET AL: "Short-chain dehydrogenease/reductase from Chloroflexus aurantiacus J-10-fl" XP002429151 gefunden im EBI accession no. Q3E5G4_CHLAU Database accession no. Q3E5G4

## Beschreibung

Die Erfindung betrifft ein Verfahren zur enantioselektiven enzymatischen Reduktion eines Hydroxyketons der allgemeinen Formel I 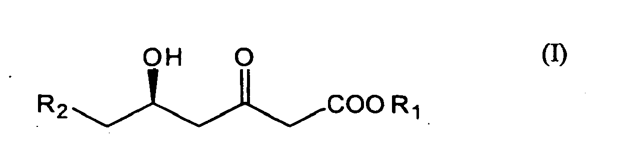 in welcher R₁ = C₁-C₆-Alkyl und R₂ = -Cl, -CN, -OH, -H oder C₁-C₆-Alkyl, zu einem chiralen Diol der allgemeinen Formel II 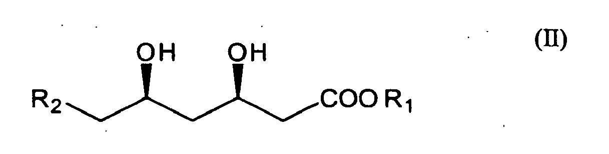 in welcher R₁ und R₂ dieselbe Bedeutung wie in Formel I besitzen, wobei das Hydroxyketon mit einer Oxidoreduktase in Gegenwart eines Cofaktors reduziert wird.

Chirale Diole der allgemeinen Formel II sind wichtige Zwischenverbindungen bei der Herstellung von Medikamenten, insbesondere bei der Herstellung von HMG-CoA-Reduktase-Inhibitoren. Solche chiralen Diole sind beispielsweise tert-Butyl (3R,5S)-6-chloro-3,5-dihydroxyhexanoat, tert-Butyl (3R,5R)-6-cyano-3,5-dihydroxyhexanoat oder tert-Butyl (5S,3R)-3,5,6-trihydroxyhexanoat.

Diole dieser Art werden insbesondere durch enantioselektive Reduktion der entsprechenden Hydroxyketone der Formel I, wie z.B tert-Butyl (5R)-6-cyano-5-hydroxy-3-oxohexanoat, tert-Butyl (5S)-6-chloro-5-hydroxy-3-oxohexanoat oder tert-Butyl (5S)-5,6-dihydroxy-3-oxohexanoat, hergestellt. Die chemisch katalysierte Reduktion hat dabei den Nachteil, dass sie einerseits aufgrund der harschen Reaktionsbedingungen zu Nebenprodukten führen kann, andererseits unbefriedigende Enantiomeren- bzw. Diastereomerenüberschüsse liefert und technisch nur sehr aufwendig durchführbar ist.

Aus diesem Grund ist man schon länger um biokatalytische Verfahren bemüht, welche die enantioselektive Reduktion der genannten Hydroxyketone erlauben. Biokatalytische Verfahren arbeiten gewöhnlich unter milden Bedingungen, weshalb zu erwarten ist, dass sie die Reduktion der ohnehin recht instabilen 5-Hydroxy-3-oxohexanoat-Derivate ohne Entstehung weiterer Nebenprodukte ermöglichen. Bislang konnten jedoch noch keine geeigneten Biokatalysatoren gefunden werden, mit denen die enzymatische Reduktion der genannten 5-Hydroxy-3-oxohexanoat-Derivate effektiv und mit isolierten Enzymen möglich ist.

Die US-Patentschrift 6,001,615 und die internationale Patentanmeldung WO 01/85975 Al beispielsweise beschreiben die Reduktion von tert-Butyl (5R)-6-cyano-5-hydroxy-3-oxohexanoat bzw. tert-Butyl (5S)-5,6-dihydroxy-3-oxohexanoat mit verschiedenen Hefen der Gattung *Beauvaria, Pichia, Candida, Kluyveromyces* und *Torulaspora.* Die Umsetzungen erfolgen dabei allerdings nur mit ganzen Zellen der Wildstämme und können daher nur in sehr geringen Konzentrationen von weit unter 5 % durchgeführt werden. Die Für die Umsetzungen verantwortlichen Enzyme und DNA-Sequenzen konnten bisher noch nicht identifiziert werden.

Mikrobielle Umsetzungen strukturell ähnlicher Verbindungen sind desweiteren in der EP 0 569 998 B1 beschrieben. Hier konnte sogar ein NADH-abhängiges Enzym aus *Acinetobacter calcoaceticus* ATCC 33305 aufgereinigt werden, welches auch isoliert gemeinsam mit Glucosedehydrogenase zur Coenzymregenerierung eingesetzt wird. Im beschriebenen Verfahren wird das Substrat in Konzentrationen von 1 % eingesetzt und eine "total turn over number" des NADH von nur 10 erreicht. Ein industriell anwendbares Verfahren wurde nicht aufgezeigt.

In der US-Patentschrift 6,645,746 B1 wird eine Aminosäuresequenz aus *Candida magnoliae* offenbart, die genutzt werden kann, um mit Hilfe von NADPH tert-Butyl (5S)-6-chloro-5-hydroxy-3-oxohexanoat zu tert-Butyl (3R,5S)-6-chloro-3,5-dihydroxyhexanoat zu reduzieren. In der Beschreibung dieses Dokuments wird das Enzym bevorzugt coexprimiert mit Glucosedehydrogenase aus *Bacillus megaterium* eingesetzt, wobei die Regenerierung des Cofaktors NADPH mit Hilfe der Glucosedehydrogenase und mit Glucose als Cosubstrat erfolgt.

Die WO 2004/111083 A beschreibt ein Verfahren zur enantioselektiven enzymatischen Reduktion von Ketonen, insbesondere 2- und 3-Oxosäureestern, wobei die Reaktion von einer Oxidoreduktase aus *Pichia capsulata* katalysiert wird.

In der WO 2005/108593 A wird ein Verfahren zur Herstellung von 1-Butanol beschrieben, bei dem 2-Butanon mit einer Carbonylreduktase, beispielsweise aus *Candida parapsilosis,* und einem Coenzym in einem Zweiphasensystem reduziert wird.

Die Erfindung stellt sich die Aufgabe, ein Verfahren bereitzustellen, das die wirtschaftlich Herstellung enantiomerenreiner Diole der allgemeinen Formel II in hoher Ausbeute und hoher Enantiomerenreinheit ohne Nebenprodukte ermöglicht.

Die oben genannte Aufgabe wird erfindungsgemäß durch ein Verfahren der eingangs genannten Art gelöst, bei welchem die Oxidoreduktase
a) eine Aminosäuresequenz aufweist, bei welcher mindestens 50% der Aminosäuren mit jenen der Aminosäuresequenz SEQ ID NO:1, SEQ ID NO:8, SEQ ID NO:11 oder SEQ ID NO:14 identisch sind, wobei die Aminosäuresequenz nicht Candida magnoliae IFO 0705 ist,
b) von der Nukleinsäuresequenz SEQ ID NO:2, SEQ ID NO:3, SEQ ID NO:9 ,SEQ ID NO:10, SEQ ID NO:12, SEQ ID NO:13 oder SEQ ID NO:15 codiert wird, oder
c) von einer Nukleinsäuresequenz codiert wird, die mit SEQ ID NO:2, SEQ ID N0:3, SEQ ID NO:9 ,SEQ ID NO:10, SEQ ID NO:12, SEQ ID NO:13 oder SEQ ID NO:15 unter stringenten Bedingungen hybridisiert.

Es wurde gefunden, dass die Polypeptide mit der Aminosäuresequenz SEQ ID NO:1, SEQ ID NO:8 und SEQ ID NO:11 Oxidoreduktaseaktivität aufweisen und verwendet werden können, um tert-Butyl (5R)-6-cyano-5-hydroxy-3-oxohexanoat zu tert-Butyl (3R,5R)-6-cyano-3,5-dihydroxyhexanoat mit einem Diastereomerenüberschuss von > 99 % zu reduzieren. In gleicher Weise können andere Hydroxyketone der allgemeinen Formel I mittels der Aminosäuresequenzen SEQ ID NO:1, SEQ ID NO:8 und SEQ ID NO:11 reduziert werden.

Zusätzlich haben die benannten Oxidoreduktasen den Vorteil, dass sie in der Lage sind, den bei der Reduktion entstandenen oxidierten Cofaktor durch Reduktion eines sekundären Alkohols zu regenerieren. Ein besonderer wirtschaftlicher Vorteil der genannten Oxidoreduktasen ist also auch darin zu sehen, dass im Gegensatz zu den Verfahren des Standes der Technik (US 6,G45,746 B und EP 0 569 998 B) kein weiteres Enzym zur Cofaktorregenerierung eingesetzt werden muss.

Eine DNA-Sequenz SEQ ID NO:2, die für ein Polypeptid mit der SEQ ID NO:1 kodiert, ist beispielsweise aus dem Genom des Organismus *Rubrobacter xylanophilus* DSM 9941 erhältlich. Außerdem wurde gefunden, dass die DNA-Sequenz SEQ ID NO:3 verwendet werden kann, um das Polypeptid der SEQ ID NO:1 in *Escherichia* zu exprimieren.

Eine DNA-Sequenz SEQ ID NO:9, die für ein Polypeptid mit der SEQ ID NO:8 kodiert, ist beispielsweise aus dem Genom des Organismus *Geobacillus thermodenitrificans* DSM 465 erhältlich. Außerdem wurde gefunden, dass die DNA-Sequenz SEQ ID NO:10 verwendet werden kann, um das Polypeptid der SEQ ID NO:8 in *Escherichia* zu exprimieren.

Eine DNA-Sequenz SEQ ID NO:12, die für ein Polypeptid mit der SEQ ID NO:11 kodiert, ist beispielsweise aus dem Genom des Organismus *Chloroflexus aurantiacus DSM 635* erhältlich.

Eine DNA-Sequenz SEQ ID NO:15, die für ein Polypeptid mit der SEQ ID NO:14 kodiert, ist beispielsweise aus dem Organismus *Candida magnoliae CBS 6396* erhältlich.

Die vorliegende Erfindung betrifft somit ein Verfahren zur Reduktion von Hydroxyketonen der allgemeinen Formel I zu Diolen der allgemeinen Formel II unter Verwendung eines Polypeptides mit der Aminosäuresequenz SEQ ID NO:1, SEQ ID NO:8, SEQ ID NO:11 oder SEQ ID NO:14, oder eines Polypeptides, das eine Aminosäuresequenz aufweist, welche mindestens zu 50 % mit der Aminosäuresequenz SEQ ID NO:1, SEQ ID NO:8, SEQ ID NO:11 oder SEQ ID NO:14 identisch ist, also eines Polypeptides, das durch Substitution, Insertion, Deletion oder Addition von mindestens einer Aminosäure von SEQ ID NO:1, SEQ ID NO:8, SEQ ID NO:11 oder SEQ ID NO:14 ableitbar ist wobei die Aminosäuresequenz nicht Candida magnoliae IF0 0705 ist, oder unter Verwendung eines Polypeptides, welches von der Nukleinsäuresequenz SEQ ID NO:2, SEQ ID NO:9, SEQ ID NO:12 oder SEQ ID NO:15 oder von einer Nukleinsäuresequenz codiert wird, welche unter stringenten Bedingungen mit SEQ ID NO:2, SEQ ID NO:9, SEQ ID NO:12 oder SEQ ID NO:15 hybridisiert.

Unter einer Nukleinsäuresequenz, welche zum Beispiel mit der SEQ ID NO:2 unter stringenden Bedingungen hybridisiert, versteht man ein Polynukleotid, welches mittels der Kolonie-Hybridisierungsmethode, der Plaque-Hybridisierungsmethode, der Southern-Hybridisierungsmethode oder Vergleichbarem unter Verwendung der SEQ ID NO:2 als DNA-Sonde identifiziert werden kann.

Zu diesem Zweck wird das an einem Filter immobilisierte Polynukleotid beispielsweise mit der SEQ ID NO:2 in einer 0,7-1 M NaCl-Lösung bei 60°C hybridisiert. Die Hybridisierung wird, wie z.B. in Molecular Cloning, A Laboratory Manual, Second Edition (Cold Spring Harbor Laboratory Press, 1989) oder ähnlichen Publikationen beschrieben, durchgeführt. Anschließend wird der Filter mit 0,1 bis 2-facher SSC-Lösung bei 65°C gewaschen, wobei unter 1-facher SSC-Lösung ein Gemisch, bestehend aus 150 mM NaCl und 15 mM Natriumcitrat, verstanden wird.

Im erfindungsgemäßen Verfahren können das Polypeptid mit der Sequenz SEQ ID NO:1, SEQ ID NO:8, SEQ ID NO:11 oder SEQ ID NO:14 bzw. von diesen Polypeptiden ableitbare Polypeptide entweder vollständig gereinigt, teilweise gereinigt oder als Zellen, enthaltend das Polypeptid SEQ ID NO:1, SEQ ID NO:8, SEQ ID NO:11 oder SEQ ID NO: 14, eingesetzt werden. Die eingesetzten Zellen können dabei nativ, permeabilisiert oder lysiert vorliegen. Bevorzugt werden das Polypeptid mit der Sequenz SEQ ID NO:1, SEQ ID NO:8, SEQ ID NO:11 oder SEQ ID NO:14 bzw. davon ableitbare Derivate in einem geeigneten Wirtsorganismus, wie beispielsweise *Escherichia coli,* überexprimiert und das rekombinante Polypeptid zur Reduktion des Hydroxyketons der allgemeinen Formel I eingesetzt.

Je kg umzusetzender Verbindung der Formel I werden 5000 bis 10 Mio U Oxidoreduktase SEQ ID NO:1, SEQ ID NO:8, SEQ ID NO:11 oder SEQ ID NO:14 bzw. deren Derivate eingesetzt (nach oben offen). Die Enzymeinheit 1 U entspricht dabei jener Enzymmenge, die benötigt wird, um 1 µmol des Hydroxyketons der Formel I je Minute (min) umzusetzen.

Die erfindungsgemäße enzymatische Reduktion läuft unter milden Bedingungen ab, so dass die Zersetzung des oft instabilen Hydroxyketons und somit die Entstehung unerwünschter Nebenprodukte weitgehend verhindert werden kann. Das erfindungsgemäßen Verfahren weist eine hohe Standzeit und eine Diastereomerenreinheit von in der Regel > 99 % der hergestellten chiralen Diole auf.

Eine bevorzugte Ausführungsform der Erfindung ist dadurch gekennzeichnet, dass der im Verfahren verwendete Cofaktor kontinuierlich mit einem Cosubstrat reduziert wird. Vorzugsweise wird als Cofactor NAD(P)H eingesetzt, wobei das bei der Reduktion gebildete NAD(P) mittels Cosubstrat wieder zu NAD(P)H reduziert wird.

Als Cosubstrat werden dabei bevorzugt sekundäre Alkohole, wie 2-Propanol, 2-Butanol, 2-Pentanol, 3-Pentanol, 4-Methyl-2-pentanol, 2-Heptanol, 2-Octanol oder Cyclohexanol eingesetzt. Gemäß einer besonders bevorzugten Ausführungsform wird 2-Propanol zur Coenzymregenerierung eingesetzt. Der Anteil des Cosubstrates für die Regenerierung kann 5 bis 95 Vol%, bezogen auf das Gesamtvolumen, betragen.

Die Coenzymregenerierung erfolgt beispielsweise ebenfalls durch das Polypeptid mit der SEQ ID NO:1, SEQ ID NO:8, SEQ ID NO:11 oder SEQ ID NO:14.

Das Hydroxyketon der allgemeinen Formel I wird im erfindungsgemäßen Verfahren vorzugsweise in einer Menge von 5 Gew.% bis 50 Gew.% (50 g/l bis 50 g/l), bezogen auf das Gesamtvolumen, eingesetzt, bevorzugt von 8 Gew.% bis 40 Gew.%, insbesondere von 10 Gew.% bis 25 Gew.%.

Eine besonders bevorzugte Ausführungsform ist dadurch gekennzeichnet, dass als Hydroxyketon der allgemeinen Formel (I) tert-Butyl (5R)-6-cyano-5-hydroxy-3-oxohexanoat, tert-Butyl (5S)-6-chloro-5-hydroxy-3-oxohexanoat oder tert-Butyl (5S)-5,6-dihydroxy-3-oxohexanoat eingesetzt wird.

Vorzugsweise wird das erfindungsgermäße Verfahren in einem wässrigen organischen Zweiphasensystem durchgeführt.

Der wässrige Anteil des Reaktionsgemisches, in dem die enzymatische Reduktion abläuft, enthält bevorzugt einen Puffer, beispielsweise Kaliumphosphat-, Tris/HCl- oder Triethanolamin-Puffer, mit einem pH-Wert von 5 bis 10, vorzugsweise pH 6 bis 9. Der Puffer kann zusätzlich noch Ionen zur Stabilisierung oder Aktivierung der Enzyme enthalten, wie beispielsweise Zinkionen oder Magnesiumionen.

Die Temperatur beträgt während der Durchführung des erfindungsgemäßen Verfahrens zweckmäßig etwa 10°C bis 70°C, bevorzugt 20°C bis 45°C.

In einer weiteren bevorzugten Ausführungsform des erfindungsgemäßen Verfahrens wird die enzymatische Umsetzung in Anwesenheit eines mit Wasser nicht oder nur beschränkt mischbaren organischen Lösungsmittels durchgeführt. Dieses Lösungsmittel ist beispielsweise ein symmetrischer oder unsymmetrischer Di(C₁-C₆)alkylether, ein geradkettiges oder verzweigtes Alkan oder Cycloalkan oder ein wasserunlöslicher sekundärer Alkohol, der zugleich das Cosubstrat darstellt. Die bevorzugten organischen Lösungsmittel sind Diethylether, tertiär-Butylmethylether, Diisopropylether, Dibutylether, Butylacetat, Heptan, Hexan, 2-Oktanol, 2-Heptanol, 4-Methyl-2-pentanol und Cyclohexanol. Das Lösungsmittel kann im Fall der letztgenannten sekundären Alkohole auch gleichzeitig als Cosubstrat zu Cofaktorregenerierung dienen.

Der Reaktionsansatz besteht beim Einsatz wasserunlöslicher Lösungsmittel bzw. Cosubstrate aus einer wässrigen und einer organischen Phase. Das Hydroxyketon verteilt sich entsprechend seiner Löslichkeit zwischen organischer und wässriger Phase. Die organische Phase hat allgemein einen Anteil von 5 bis 95%, bevorzugt von 10 bis 90%, bezogen auf das gesamte Reaktionsvolumen. Die zwei flüssigen Phasen werden bevorzugt mechanisch gemischt, so dass eine große Oberfläche zwischen ihnen erzeugt wird. Auch in dieser Ausführungsform kann z.B. das bei der enzymatischen Reduktion gebildete NAD mit einem Cosubstrat, wie oben beschrieben, wieder zu NADH reduziert werden.

Die Konzentration des Cofaktors, insbesondere NADH bzw. NADPH, in der wässrigen Phase beträgt allgemein 0,001 mM bis 10 mM, insbesondere 0,01 mM bis 1 mM.

Im erfindungsgemäßen Verfahren kann auch ein Stabilisator der Oxidoreduktase/Dehydrogenase eingesetzt werden. Geeignete Stabilisatoren sind beispielsweise Glycerin, Sorbitol, 1,4-DL-Dithiothreit (DTT) oder Dimethylsulfoxid (DMSO).

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt.

Gemäß einer weiteren möglichen Ausführungsform der Erfindung kann das oxidierte Cosubstrat (beispielsweise Aceton) kontinuierlich entfernt werden und/oder das Cosubstrat (beispielsweise 2-Propanol) kontinuierlich neu zugeführt werden, um das Reaktionsgleichgewicht in Richtung des Reaktionsproduktes (Diol der allgemeinen Formel II) zu verschieben.

In einer weiteren Ausführungsform kann die Zugabe der Oxidoereduktase gemäß SEQ ID NO:1, SEQ ID NO:8, SEQ ID NO:11 oder SEQ ID NO:14 und/oder des Cosubstrates im Prozessverlauf auch schrittweise erfolgen.

Nach Beendigung der Reduktion wird das Reaktionsgemisch aufgearbeitet. Dazu wird die wässrige Phase gegebenenfalls von der organischen Phase abgetrennt und die das Produkt enthaltende organische Phase filtriert. Die wässrige Phase kann gegebenenfalls auch extrahiert und wie die organische Phase weiter aufgearbeitet werden. Danach wird das Lösungsmittel aus der organischen Phase verdampft und das Diol der allgemeinen Formel II als Rohprodukt erhalten. Das Rohprodukt kann dann weiter aufgereinigt oder direkt für die Synthese eines Folgeproduktes verwendet werden.

Die Erfindung wird nachstehend anhand von Beispielen weiter erläutert.

### Beispiel 1

### Klonierung einer Oxidoreduktase aus Rubrobacter xylanophilus DSM 9941

### A) Anzucht von Rubrobacter xylanophilus DSM 9941

Zellen von *Rubrobacter xylanophilus* DSM 9941 wurden in folgendem Medium bei 50°C (pH 7,2) und 140 rpm in einem Bakterien-Schüttler kultiviert: 0,1 % Hefeextrakt, 0,1 % Trypton, 0,004 % CaSO₄ x 2 H₂O, 0,02 % MgCl₂ x 6 H₂O, 0,01 % Nitrilotriessigsäure, 100 ml Phosphat-Puffer [5,44 g/l KH₂PO₄, 43 g/l Na₂HPO₄ x 12 H₂O], 500 µl/l 0,01 M Fe-Citrat, 500 µl/l Trace Element [500 µl/l H₂SO₄, 2,28 g/l MnSO₄ x H₂O, 500 mg/l ZnSO₄ x 7 H₂O, 500 mg H₃BO₃, 25 mg/l CuSO₄ x 5 H₂O, 25 mg/l Na₂MoO₄ x 2 H₂O, 45 mg/l CoCl₂ x 6 H₂O]. Am Tag 6 der Kultivierung wurden Zellen mittels Zentrifugation vom KulturMedium separiert und bei -80°C gelagert.

### B) Amplifikation des für selektive Oxidoreduktase kodierenden Gens

Genomische DNA wurde nach der in ,,Molecular Cloning" von Manniatis & Sambrook beschriebenen Methode extrahiert. Die resultierende Nukleinsäure diente als Matrize für die Polymerase-Kettenreaktion (PCR) mit spezifischen Primern, die von der in der NCBI-Datenbank unter der Nummer 46106817 publizierten Gen-Sequenz abgeleitet wurden. Dabei wurden die Primer für eine nachfolgende Klonierung in einen Expressionsvektor 5'-terminal mit Restriktionsschnittstellen für die Endonukleasen Nde I und Hind III, bzw. Sph I versehen (SEQ ID NO:4, SEQ ID NO:5, SEQ ID NO:7).

Die Amplifizierung wurde in einem PCR-Puffer [10 mM Tris-HCl, (pH 8,0); 50 mM KCl; 10 mM MgSO₄; 1 mM dNTP Mix; je 20 pMol Primer und 2,5 U Platinum Pfx DNA-Polymerase (Invitrogen)] mit 500 ng genomischer DNA und folgenden Temperatur-Zyklen durchgeführt:

| | |
|---|---|
| Zyklus 1 : | 94°C, 2 min |
| Zyklus 2 x 30: | 94°C, 15 sec |
| | 54°C, 30 sec |
| | 68°C, 60 sec |
| Zyklus 3: | 68°C, 7 min |
| | 4°C, ∞ |

Das resultierende PCR-Produkt mit einer Größe von etwa 750 bp wurde nach der Reinigung über ein 1 % Agarose-Gel mit Hilfe der Endonukleasen *Nde I* und *Hind III* bzw. *Sph I* und *Hind III* restringiert und in das mit den gleichen Endonukleasen behandelte Rückgrad des pET21a Vectors (Novagen) bzw. pQE70 Vectors (Qiagen) ligiert. Nach der Transformation von 2 µl des Ligationsansatzes in *E. coli* Top 10 F' Zellen (Invitrogen) wurde Plasmid-DNA Ampicillin-resistenter Kolonien mittels einer Restriktionsanalyse mit den Endonukleasen *Nde I* und *Hind III* bzw. *Sph I* und *Hind III* auf das Vorhandensein eines 750 bp großen Insert getestet. Plasmid-Präparationen aus den für das Fragment positiven Klonen wurden einer Sequenzanalyse unterzogen und anschließend in *Escherichia coli* BL21 Star (Invitrogen) bzw. *E. coli* RB791 (genetic stock, Yale) transformiert.

### Beispiel 2

### Effiziente Expression des Polypeptides SEQ ID NO:1 in Escherichia coli-Zellen

Für die effiziente Expression des Polypeptides SEQ ID NO:1 in *Escherichia coli-*Zellen wurde kodierende DNA SEQ ID NO:3 für die Klonierung in einen Expressionsvektor als Matrize in einer PCR-Reaktion verwendet. Diese DNA-Sequenz unterschied sich im Bereich der ersten 160 Basenpaare von der zuvor bekannten DNA-Sequenz (SEQ ID NO:2) in 51 Basen. Diese Modifizierung war konservativ und führte nicht zur Veränderung der Aminosäuresequenz.

Die Amplifizierung wurde in einem PCR-Puffer [10 mM Tris-HCl, (pH 8,0); 50 mM KCl; 10 mM MgSO₄; 1 mM dNTP Mix; je 20 pMol Primer (SEQ ID NO:6, SEQ ID NO:5) und 2,5 U Platinum Pfx DNA-Polymerase (Invitrogen)] mit 50 ng DNA SEQ ID NO:3 als Matrize und folgenden Temperatur-Zyklen durchgeführt:

| | |
|---|---|
| Zyklus 1: | 94°C, 2 min |
| Zyklus 2 x 30: | 94°C, 40 sec |
| | 56°C, 30 sec |
| | 68°C, 60 sec |
| Zyklus 3: | 68°C, 7 min |
| | 4°C, ∞ |

Das resultierende PCR-Produkt mit einer Größe von etwa 750 bp wurde nach der Reinigung über ein 1 % Agarose-Gel mit Hilfe der Endonukleasen *Nhe I* und *Hind III* in das mit den gleichen Endonukleasen behandelte Rückgrad des pET21a Vectors (Novagen) ligiert. Nach der Transformation von 2 µl des Ligationsansatzes in *E. coli* Top 10 F' Zellen (Invitrogen) wurde Plasmid-DNA Ampicillin-resistenter Kolonien mittels einer Restriktionsanalyse mit den Endonukleasen *Nhe I* und *Hind III* auf das Vorhandensein eines 750 bp großen Inserts getestet. Plasmid-Präparationen aus den für das Fragment positiven Klonen wurden einer Sequenzanalyse unterzogen und anschließend in *Escherichia coli* BL21 Star (Invitrogen), transformiert.

### Beispiel 3

### Herstellung der Oxidoreduktase aus Rubrobacter xylanonhilus DSM 9941

Die mit dem Expressionskonstrukt transformierten *Escherichia coli-Stämme* BL21 Star (Invitrogen, Karlsruhe, Deutschland), bzw. RB791 (*E. coli* genetic stock, Yale, USA) wurden in Medium (1% Tryptone, 0,5% Hefeextrakt, 1% NaCl) mit Ampicillin (50 µg/ml), kultiviert, bis eine optische Dichte von 0,5, gemessen bei 550 nm, erreicht war. Die Expression von rekombinantem Protein wurde durch Zugabe von Isopropylthiogalaktosid (IPTG) in einer Konzentration von 0,1 mM induziert. 16 Stunden nach der Induktion bei 25°C und 220 rpm wurden die Zellen geerntet und bei -20°C eingefroren.

Zur Enzymgewinnung wurden 30 g Zellen in 150 ml Triethanolaminpuffer (100 mM, pH = 7, 2 mM MgCl₂, 10% Glycerin) suspendiert und mittels Hochdruckhomogenisator aufgeschlossen. Die Enzymlösung wurde anschließend mit 150 ml Glycerin versetzt und bei -20°C gelagert.

Die derart erhaltene Enzymlösung wurde zur Synthese von tert-Butyl (3R,5R)-6-cyano-3,5-dihydroxyhexanoat eingesetzt.

Die erhaltene Enzymlösung wurde verdünnt auch für die entsprechenden enzymatischen Messungen eingesetzt. Der Aktivitätstest setzte sich dabei wie folgt zusammen: 870 µl 100 mM TEA-Puffer, pH 7,0, 160 µg NAD(P)H, 10 µl verdünntes Zelllysat. Die Reaktion wurde durch Zugabe von 100 µl der 100 mM Substratlösung tert-Butyl (5R)-6-cyano-5-hydroxy-3-oxohexanoat zum Reaktionsgemisch gestartet.

### Beispiel 4

### Umsetzung von tert-Butyl (5R)-6-Cyano-5-hydroxy-3-oxohexanoat zu tert-Butyl (3R,5R)-6-cyano-3,5-dihydroxyhexanoat mittels Oxidoreduktase SEQ ID NO:1

Für die Umsetzung von tert-Butyl (5R)-6-cyano-5-hydroxy-3-oxohexanoat zu term-butyl (3R,5R)-6-cyano-3,5-dihydroxyhexanoat wurde im Eppendorfreaktionsgefäß ein Gemisch aus 900 µl Puffer (100 mM TEA, pH = 7, 1 mM MgCl₂), 100 µl 2-Propanol, 10 µl tert-Butyl (5R)-6-cyano-5-hydroxy-3-oxohexanoat-Rohprodukt (Enantiomerenreinheit >99%), 0,1 mg NAD und 100 µl Enzymsuspension (siehe Beispiel 3) für 24 h bei Raumtemperatur unter ständiger Durchmischung inkubiert. Nach 24 h waren 96% des eingesetzten tert-Butyl (5R)-6-cyano-5-hydroxy-3-oxohexanoat zu tert-Butyl (3R,5R)-6-cyano-3,5-dihydroxyhexanoat reduziert. Der Diastereomerenüberschuss betrug>99%.

Die Bestimmung des Umsatzes sowie des Diastereomerenüberschusses erfolgte mittels chiraler Gas-Chromatographie. Dazu wurde ein Gas-Chromatograph GC-17A von Shimadzu mit einer chiralen Trennsäule CP-Chirasil-DEX CB (Varian Chrompack, Darmstadt, Deutschland), einem Flammen-Ionisations-Detektor und Helium als Trägergas benutzt.

Die Trennung von tert-Butyl-6-cyano-3,5-dihydroxyhexanoat erfolgte bei 0,72 bar und 10 min bei 50°C, 5°C/min → 200°C für 10 min.

Die Retentionszeiten betrugen: (R-BCH) 4,4 min; (R,R-BCH) 47,1 min und (R,S-BCH) 48,2 min.

### Beispiel 5

### Synthese von tert-Butyl (3R,5R)-6-cyano-3,5-dihydroxyhexanoat aus tert-Butyl (5R)-6-cyano-5-hydroxy-3-oxohexanoat mittels Oxidoreduktase SEQ ID NO:1

Für eine weitere Umsetzung des tert-Butyl (5R)-6-cyano-5-hydroxy-3-oxohexanoat zu tert-Butyl (3R,5R)-6-cyano-3,5-dihydroxyhexanoat wurde im Eppendorfreaktionsgefäß ein Gemisch aus 550 µl Puffer (100 mM TEA, pH = 7, 1 mM MgCl₂), 150 µl 2-Propanol, 200 µl tert-Butyl (5R)-6-cyano-5-hydroxy-3-oxohexanoat-Rohprodukt (Enantiomerenreinheit >99%), 0,1 mg NAD und 200 µl Enzymsuspension (siehe Beispiel 3) inkubiert. Im Verlauf der Reaktion wurde das entstandene Aceton/2-Propanolgemisch mehrfach durch Einleiten von Stickstoff verdampft und frisches 2-Propanol und 50 µl Enzym wurden zugegeben. Nach 2- bis 3-facher Wiederholung im Abstand von 24 h waren >90% des eingesetzten tert-Butyl (5R)-6-cyano-5-hydroxy-3-oxohexanoat zu tert-Butyl (3R,5R)-6-cyano-3,5-dihydroxyhexanoat reduziert. Der Diastereomerenüberschuss betrug >99%.

### Beispiel 6

### Synthese von tert-Butyl (3R,5R)-6-cyano-3,5-dihydroxyhexanoat aus tert-Butyl (5R)-6-cyano-5-hydroxy-3-oxohexanoat mittels Oxidoreduktase SEQ ID NO:1

Für eine weitere Umsetzung des tert-Butyl (5R)-6-cyano-5-hydroxy-3-oxohexanoat zu tert-Butyl (3R,5R)-6-cyano-3,5-dihydroxyhexanoat wurde in einem Reaktiongefäß ein Gemisch aus 6,7 ml Puffer (100 mM TEA, pH = 9), 1,7 ml 2-Propanol, 2 ml tert-Butyl (5R)-6-cyano-5-hydroxy-3-oxohexanoat-Rohprodukt (Enantiomerenreinheit >99%), 1,0 mg NAD und 150 mg gefrorene Zellen *E. coli* BL21 Star, enthaltend die Oxidoreduktase SEQ ID NO:1, (siehe Beispiel 3) bei 45°C inkubiert. Nach 24 h waren > 90 % des eingesetzten tert-Butyl (5R)-6-cyano-5-hydroxy-3-oxohexanoat zu tert-Butyl (3R,5R)-6-cyano-3,5-dihydroxyhexanoat reduziert. Der Diastereomerenüberschuss betrug >99%.

### Beispiel 7

### Synthese von tert-Butyl (3R,5R)-6-cyano-3,5-dihydroxyhexanoat aus tert-Butyl (5R)-6-cyano-5-hydroxy-3-oxohexanoat mittels Oxidoreduktase SEQ ID NO:8

Für eine weitere Umsetzung des tert-Butyl (5R)-6-cyano-5-hydroxy-3-oxohexanoat zu tert-Butyl (3R,5R)-6-cyano-3,5-dihydroxyhexanoat wurde in einem Reaktiongefäß ein Gemisch aus 5,0 ml Puffer (100 mM TEA, pH = 7,5), 2,0 ml 2-Propanol, 4,0 ml tert-Butyl (5R)-6-cyano-5-hydroxy-3-oxohexanoat-Rohprodukt (Enantiomerenreinheit >99%), 1,0 mg NAD und 250 mg gefrorene Zellen *E. coli* RB 791, enthaltend die Oxidoreduktase SEQ ID NO: 8, (entsprechend Beispiel 2 und 3) bei 40°C inkubiert. Nach 24 h waren >90% des eingesetzten tert-Butyl (5R)-6-cyano-5-hydroxy-3-oxohexanoat zu tert-Butyl (3R,5R)-6-cyano-3,5-dihydroxyhexanoat reduziert. Der Diastereomerenüberschuss betrug >99%.

### Beispiel 8

### Synthese von tert-Butyl (3R,5R)-6-cyano-3,5-dihydroxyhexanoat aus tert-Butyl (5R)-6-cyano-5-hydroxy-3-oxohexanoat mittels Oxidoreduktase SEQ ID NO:11

Für die Umsetzung von tert-Butyl (5R)-6-cyano-5-hydroxy-3-oxohexanoat zu tert-Butyl (3R,5R)-6-cyano-3,5-dihydroxyhexanoat wurde im Eppendorfreaktionsgefäß ein Gemisch aus 350 µl Puffer (100 mM Kaliumphosphat, pH = 7), 150 µl 2-Propanol, 50 µl tert-Butyl (5R)-6-cyano-5-hydroxy-3-oxohexanoat-Rohprodukt (Enantiomerenreinheit >99%), 0,025 mg NAD und 15 µl Enzymsuspension SEQ ID NO:11 (siehe Beispiel 3) für 48 h bei Raumtemperatur unter ständiger Durchmischung inkubiert. Nach 48 h waren >80% des eingesetzten tert-Butyl (5R)-6-cyano-5-hydroxy-3-oxohexanoat zu tert-Butyl (3R,5R)-6-cyano-3,5-dihydroxyhexanoat reduziert. Der Diastereomerenüberschuss betrug >99%.

### Sequenzliste

***SEQ ID NO:1***
***SEQ ID NO:2***
***SEQ ID NO:3***
***SEQ ID NO:4***
   GGGAATTCCATATGATGCTCGAGGGGAAGGTCG
***SEQ ID NO:5***
   CCCAAGCTTATTACTTGAACTCCGCGTAGCCGTC
***SEQ ID NO:6***
   CCTAGCTAGCATGCTGGAAGGTAAAGTGGC
***SEQ ID NO:7***
   CACATGCATGCGAATGCTCGAGGGGAAGGTC
***SEQ ID NO:8***
***SEQ ID NO:9***
***SEQ ID NO:10***
***SEQ ID NO:11***
***SEQ ID NO:12***
***SEQ ID NO:13***
***SEQ ID NO:14***
***SEQ ID NO:15***

## Patentansprüche

1. Verfahren zur enantioselektiven enzymatischen Reduktion eines Hydroxyketons der allgemeinen Formel 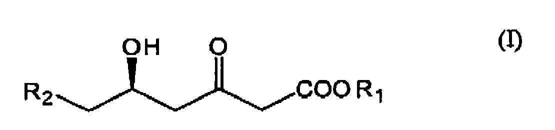 in welcher R₁ = C₁-C₆-Alkyl und R₂ =-Cl, -CN, -OH, -H oder C₁-C₆-Alkyl,
zu einem chiralen Diol der allgemeinen Formel II 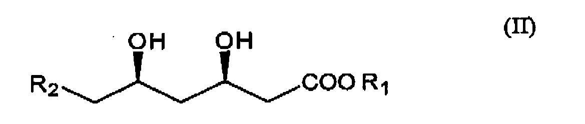 in welcher R₁ und R₂ dieselbe Bedeutung wie in Formel I besitzen,
wobei das Hydroxyketone mit einer Oxidoreduktase in Gegenwart eines Cofakrtors reduziert wird, **dadurch gekennzeichnet, dass** die Oxidoreduktase
a) eine Aminosäuresequenz aufweist, bei welcher mindestens 50% der Aminosäuren mit jenen der Aminosäuresequenz SEQ ID NO*:*1, SEQ ID NO:8, SEQ ID NO:11 oder SEQ ID NO:14 identisch sind, wobei die Aminosäuresequenz nicht *Candida magnoliae* IFO 0705 ist,
b) von der Nukleinsäuresequenz SEQ ID NO:2, SEQ ID NO:3, SEQ ID NO:9, SEQ ID NO:10, SEQ ID NO:12, SEQ ID NO:13 oder SEQ ID NO:15 codiert wird, oder
c) von einer Nukleinsäuresequenz codiert wird, die mit SEQ ID NO:2. SEQ ID NO:3, SEQ ID NO:9 ,SEQ ID NO:10, SEQ ID NO:12, SEQ ID NO:13 oder SEQ ID NO:15 unter stringenten Bedingungen hybridisiert.

2. Verfahren nach Anspruch 1, **dadurch gekennzeichnet, dass** der Cofaktor kontinuierlich mit einem Cosubstrat reduziert wird.

3. Verfahren nach Anspruch 1 oder 2, **dadurch gekennzeichnet, dass** als Cofaktor NAD(P)H eingesetzt wird.

4. Verfahren nach einem der Ansprüche 1 bis 3, **dadurch gekennzeichnet, dass** als Cosubstrat bzw. sekundärer Alkohol 2-Propanol, 2-Butanol, 2-Pentanol, 4-Methyl-2-pentanol, 2-Heptanol oder 2-Octanol eingesetzt wird.

5. Verfahren nach einem der Ansprüche 1 bis 4, **dadurch gekennzeichnet, dass** das Hydroxyketon in einer Menge von 5-50 Gew.%, vorzugsweise 8-40 Gew.%, insbesondere 10-25 Gew.%, bezogen auf das Gesamtreaktionsvolumen, eingesetzt wird.

6. Verfahren nach einem der Ansprüche 1 bis, 5, **dadurch gekennzeichnet, dass** als Hydroxyketon der allgemeinen Formel (I) tert-Butyl (5R)-6-cyano-5-hydroxy-3-oxohexanoat, tert-Butyl (5S)-6-chloro-5-hydroxy-3-oxohexanoat oder tert-Butyl (5S)-5,6-dihydroxy-3-oxohexanoat eingesetzt wird.

7. Verfahren nach einem der Ansprüche 1 bis 6, **dadurch gekennzeichnet, dass** der TTN (total turn over number = mol reduziertes Hydroxyketon/ mol eingesetzter Cofactor) ≥ 10³ ist.

8. Verfahren nach einem der Ansprüche 1 bis, 7, **dadurch gekennzeichnet, dass** es in einem wässrigen organischen Zweiphasensystem durchgeführt wird.

9. Verfahren nach einem der Ansprüche 1 bis 8, **dadurch gekennzeichnet, dass** zusätzlich ein organisches Lösungsmittel, wie Diethylether, tertiär-Butylmethylether, Diisopropylether, Dibutylether, Ethylacetat, Butylacetat, Heptan, Hexan oder Cyclohexan, einsetzt wird.

## Claims

1. A method for the enantioselective enzymatic reduction of a hydroxy ketone of general formula I 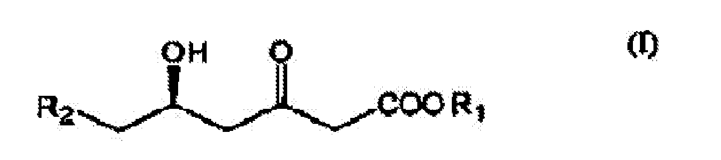 wherein R₁ = C₁-C₆-alkyl and R₂ = -Cl, -CN, -OH, -H or C₁-C₆-alkyl to a chiral diol of general formula II 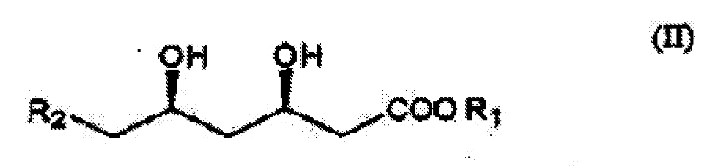 with R₁ and R₂ having the same meaning as in formula I,
wherein the hydroxy ketone is reduced with an oxidoreductase in the presence of a co-factor, **characterized in that** the oxidoreductase
a) has an amino acid sequence, wherein at least 50% of the amino acids are identical to those of the amino acid sequence SEQ ID NO: 1, SEQ ID NO: 8, SEQ ID NO: 11 or SEQ ID NO: 14, wherein the amino acid sequence is not *Candida magnoliae* IFO 0705,
b) is encoded by the nucleic acid sequence SEQ ID NO: 2, SEQ ID NO: 3, SEQ ID NO: 9, SEQ ID NO: 10, SEQ ID NO: 12, SEQ ID NO: 13 or SEQ ID NO: 15, or
c) is encoded by a nucleic acid sequence which hybridizes with SEQ ID NO: 2, SEQ ID NO: 3, SEQ ID NO: 9, SEQ ID NO: 10, SEQ ID NO: 12, SEQ ID NO: 13 or SEQ ID NO: 15 under stringent conditions.

2. The method according to claim 1, **characterized in that** the co-factor is continuously being reduced with a co-substrate.

3. The method according to claim 1 or 2, **characterized in that** NAD(P)H is used as the co-factor.

4. The method according to any of claims 1 to 3, **characterized in that** 2-propanol, 2-butanol, 2-pentanol, 4-methyl-2-pentanol, 2-heptanol or 2-octanol is used as the co-substrate and secondary alcohol, respectively.

5. The method according to any of claims 1 to 4, **characterized in that** the hydroxy ketone is used in an amount of 5 to 50 % by weight, preferably 8 to 40 % by weight, in particular 10 to 25 % by weight, based on the total reaction volume.

6. The method according to any of claims 1 to 5, **characterized in that** tert.-butyl (5R)-6-cyano-5-hydroxy-3-oxohexanoate, tert.-butyl (5S)-6-chloro-5-hydroxy-3-oxohexanoate or tert.-butyl (5S)-5,6-dihydroxy-3-oxohexanoate is used as the hydroxy ketone of general formula (I).

7. The method according to any of claims 1 to 6, **characterized in that** the TTN (total turn over number = mol of reduced hydroxy ketone/mol of used co-factor) is ≥ 10³.

8. The method according to any of claims 1 to 7, **characterized in that** it is carried out in an aqueous organic two-phase-system.

9. The method according to any of claims 1 to 8, **characterized in that** additionally an organic solvent such as diethyl ether, tert.-butyl methyl ether, diisopropyl ether, dibutyl ether, ethyl acetate, butyl acetate, heptane, hexane or cyclo hexane is used.

## Revendications

1. Procédé de réduction enzymatique éniantosélective d'une hydroxycétone de formule générale I 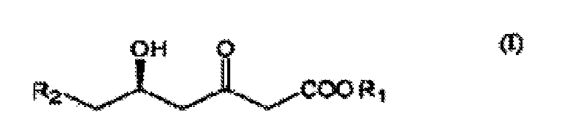 dans laquelle R₁ = alkyle en C₁ à C₆ et R₂ = -Cl, -CN, -OH, -H ou un alkyle en C₁ à C₆,
en un diol chiral de formule générale II 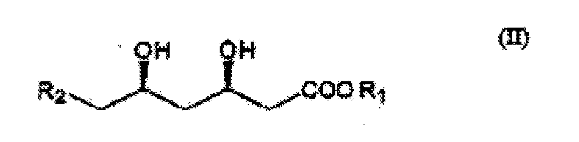 dans laquelle R₁ et R₂ ont le même sens que dans la formule I,
l'hydroxycétone étant réduite à l'aide d'une oxydoréductase en présence d'un cofacteur,
**caractérisé en ce que**
l'oxydoréductase
a) présente une séquence d'acides aminés dont au moins 50 % des acides aminés sont identiques à ceux des séquences d'acides aminés SEQ ID NO : 1, SEQ ID NO : 8, SEQ ID NO : 11 ou SEQ ID NO : 14, la séquence d'acide aminés n'étant pas *Candida magnoliae* IFO 0705,
b) est codée par les séquences d'acides nucléiques SEQ ID NO : 2, SEQ ID NO : 3, SEQ ID NO : 9, SEQ ID NO : 10, SEQ ID NO : 12, SEQ ID NO : 13 ou SEQ ID NO : 15 ou
c) est codée par une séquence d'acides nucléiques qui s'hybride avec les SEQ ID NO : 2, SEQ ID NO : 3, SEQ ID NO : 9, SEQ ID NO : 10, SEQ ID NO : 12, SEQ ID NO : 13 ou SEQ ID NO : 15 dans des conditions stringentes.

2. Procédé selon la revendication 1, **caractérisé en ce que** le cofacteur est réduit de manière continue avec un cosubstrat.

3. Procédé selon les revendications 1 ou 2, **caractérisé en ce qu'**on utilise le NAD(P)H comme cofacteur.

4. Procédé selon l'une des revendications 1 à 3, **caractérisé en ce qu'**on utilise comme cosubstrat ou alcool secondaire le 2-propanol, le 2-butanol, le 2-pentanol, le 4-méthyl-2-pentanol, le 2-heptanol ou le 2-octanol.

5. Procédé selon l'une des revendications 1 à 4, **caractérisé en ce qu'**on utilise de l'hydroxycétone à une concentration de 5 à 50 % en poids, de préférence de 8 à 40 % en poids et en particulier de 10 à 25 % en poids par rapport au volume total de réaction.

6. Procédé selon l'une des revendications 1 à 5, **caractérisé en ce qu'**on utilise comme hydroxycétone de formule générale (I) le (5R)-6-cyano-5-hydroxy-3-oxohexanoate de tert.-butyle, le (5S)-6-chloro-5-hydroxy-3-oxohexanoate de tert.-butyle ou le (5S)-5,6-dihydroxy-3-oxohexanoate de tert.-butyle.

7. Procédé selon l'une des revendications 1 à 6, **caractérisé en ce que** le TTN ("total turn over number" - nombre total de renouvellement = mol d'hydroxycétone réduite/mol de cofacteur consommé) est ≥ 10³.

8. Procédé selon l'une des revendications 1 à 7, **caractérisé en ce qu'**il est conduit dans un système aqueux organique biphasique.

9. Procédé selon l'une des revendications 1 à 8, **caractérisé en ce qu'**on utilise en plus un solvant organique, comme le diéthyléther, le méthyléther de butyl tertiaire, le diisopropyléther, le dibutyléther, l'acétate d'éthyle, l'acétate de butyle, l'heptane, l'hexane ou la cyclohexanone.


### SEQUENCE LISTING

<110> IEP GmbH
<120> Verfahren zur enantioselektiven enzymatischen Reduktion von Hydroxyketoverbindungen
<130> I 9844
<150> AT A 2027/2005
   <151> 2005-12-19
<160> 15
<170> PatentIn version 3.3
<210> 1
   <211> 249
   <212> PRT
   <213> Rubrobacter xylanophilus DSM 9941
<400> 1
   Met Leu Glu Gly Lys Val Ala Val Ile Thr Gly Ala Gly Ser Gly Ile
   1 5 10 15
   Gly Arg Ala Thr Ala Leu Lys Phe Ala Arg Glu Gly Ala Arg Val Val
   20 25 30
   Ala Ala Glu Leu Asp Glu Arg Gly Gly Glu Gly Val Val Arg Glu Val
   35 40 45
   Arg Ser Leu Gly Gly Glu Ala Val Phe Val Arg Thr Asp Val Ser Glu
   50 55 60
   Phe Ala Gln Val Glu Asp Ala Val Glu Arg Ala Val Gly Glu Tyr Gly
   65 70 75 80
   Thr Leu Asp Val Met Phe Asn Asn Ala Gly Ile Gly His Tyr Ala Pro
   85 90 95
   Leu Leu Glu His Glu Pro Glu His Tyr Asp Arg Val Val Arg Val Asn
   100 105 110
   Gln Tyr Gly Val Tyr Tyr Gly Ile Leu Ala Ala Gly Arg Lys Met Val
   115 120 125
   Ala Leu Lys Asn Pro Gly Leu Ile Ile Asn Thr Ala Ser Val Tyr Ala
   130 135 140
   Phe Leu Ala Ser Pro Gly Val Ile Gly Tyr His Ala Ala Lys Gly Ala
   145 150 155 160
   Val Lys Met Met Thr Gln Ala Ala Ala Leu Glu Leu Ala Pro His Gly
   165 170 175
   Ile Arg Val Val Ala Ile Ala Pro Gly Gly Val Asp Thr Pro Ile Ile
                   180                 185                 190
   Gln Gly Tyr Lys Asp Met Gly Leu Gly Glu Arg Leu Ala Arg Gly Gln
           195                 200                 205
   Met Arg Arg Arg Leu Gln Thr Pro Glu Gln Ile Ala Gly Ala Val Ala
       210                 215                 220
   Leu Leu Ala Thr Asp Glu Ala Asp Ala Ile Asn Gly Ser Val Val Met
   225                 230                 235                 240
   Thr Asp Asp Gly Tyr Ala Glu Phe Lys
                   245
<210> 2
   <211> 750
   <212> DNA
   <213> Rubrobacter xylanophilus DSM 9941
<400> 2
   atgctcgagg ggaaggtcgc ggtcatcacg ggggccggaa gcggcatagg ccgggccacc      60
   gcgctcaagt tcgcccgcga gggggcccgg gtcgtcgccg ccgagctcga cgagcgcggc     120
   ggggaggggg tggtccggga ggtgcgcagc ctcgggggcg aggcggtctt cgtccggacc     180
   gacgtctcgg agttcgcgca ggtggaggac gccgtcgagc gggcggtcgg ggagtacggc     240
   accctcgacg tgatgttcaa caacgccggc atcgggcact acgcccccct gctggagcac     300
   gagcccgagc actacgaccg ggtggtccgg gtgaaccagt acggcgtcta ctacgggata     360
   ctcgccgccg ggagaaagat ggtcgccctg aagaaccccg gcttgatcat caacaccgcc     420
   tcggtctacg ccttcctcgc ctcgccgggg gtcatcggct accacgccgc caagggggcg     480
   gtcaagatga tgacccaggc ggcggcgctg gagctcgccc cgcacggcat aagggtcgtc     540
   gccatcgccc cgggcggggt ggacaccccc atcatccagg gctacaagga catggggctc     600
   ggcgagaggc tggcccgcgg ccagatgcgc cgccggctcc agacccccga gcagatcgcc     660
   ggggcggtcg ccctgctcgc caccgacgag gccgacgcca taaacggctc ggtggtcatg     720
   accgacgacg gctacgcgga gttcaagtag                                      750
<210> 3
   <211> 750
   <212> DNA
   <213> Synthetic construct
<400> 3
   atgctggaag gtaaagtggc agtcatcacc ggtgcaggca gcggcattgg gcgtgccact      60
   gcgctgcgtt ttgcgcgtga aggcgctcgc gtcgttgtgg ccgagctgga tgaacgtcgc     120
   ggtgaggaag ttgtacgtga gattctggaa tccggtggcg aagccgtgtt tgtccgcacc     180
   gacgtgtctg aattcgagca agtcgaagcg gcagttgaac gtgcagtcga agagtacggc     240
   accctggatg tgatgttcaa caatgcgggt attgggcatt acgctccgct gttagaacat     300
   gacccagagc actatgatcg cgtggtacgt gtaaatcagt atggtgtgta ttacggaatc     360
   ctggcagccg gccgtaaaat ggccgaactg gaaaatccgg gcgttatcat taacaccgca     420
   agtgtgtacg cgtttctggc tagcccgggg gtgatcggct accatgcctc aaagggtgct     480
   gtgaaaatga tgacgcaggc tgcggccctg gaattggctc cgcacggcat ccgagttgtc     540
   gcgatcgccc cggggggcgt cgacacccct attatccaag gttataaaga tatgggcctg     600
   ggtgaacgct tggcgcgtgg tcagatgcgg cgtcgcctgc agactccgga acagattgcg     660
   ggtgcagttg tgttactggc taccgaagag gcggacgcga ttaacggttc ggtcgttatg     720
   actgatgatg ggtatgcgga gtttaaataa                                      750
<210> 4
   <211> 33
   <212> DNA
   <213> synthetic construct
   <400> 4
   gggaattcca tatgatgctc gaggggaagg tcg 33
<210> 5
   <211> 34
   <212> DNA
   <213> synthetic construct
<400> 5
   cccaagctta ttacttgaac tccgcgtagc cgtc 34
<210> 6
   <211> 30
   <212> DNA
   <213> Synthetic construct
<400> 6
   cctagctagc atgctggaag gtaaagtggc 30
<210> 7
   <211> 31
   <212> DNA
   <213> synthetic construct
<400> 7
   cacatgcatg cgaatgctcg aggggaaggt c 31
<210> 8
   <211> 250
   <212> PRT
   <213> Geobacillus thermodenitrificans DSM 465
<400> 8
   Met Arg Leu Lys Gly Lys Ala Ala Ile Val Thr Gly Gly Ala Ser Gly
   1               5                   10                  15
   Ile Gly Arg Ala Thr Ala Ile Arg Phe Ala Glu Glu Gly Ala Lys Val
               20                  25                  30
   Ala Val Ser Asp Ile Asn Glu Glu Gly Gly Glu Glu Thr Val Arg Leu
           35                  40                  45
   Ile Arg Glu Lys Gly Gly Glu Ala Ile Phe Val Gln Thr Asp Val Ala
       50                  55                  60
   Asp Ser Lys Gln Val Ser Arg Leu Val Gln Thr Ala Val Asp Ala Phe
   65                  70                  75                  80
   Gly Gly Leu His Ile Leu Phe Asn Asn Ala Gly Ile Gly His Ser Glu
                   85                  90                  95
   Val Arg Ser Thr Asp Leu Ser Glu Glu Glu Trp Asp Arg Val Ile Asn
               100                 105                 110
   Val Asn Leu Lys Gly Val Phe Leu Gly Ile Lys Tyr Ala Val Pro Val
           115                 120                 125
   Met Lys Gln Cys Gly Gly Gly Ala Ile Val Asn Thr Ser Ser Leu Leu
       130                 135                 140
   Gly Ile Lys Gly Lys Lys Tyr Glu Ser Ala Tyr Asn Ala Ser Lys Ala
   145                 150                 155                 160
   Gly Val Ile Leu Leu Thr Lys Asn Ala Ala Leu Glu Tyr Gly Lys Phe
                   165                 170                 175
   Asn Ile Arg Val Asn Ala Ile Ala Pro Gly Val Ile Asp Thr Asn Ile
               180                 185                 190
   Ile Thr Pro Trp Lys Gln Asp Glu Arg Lys Trp Pro Ile Ile Ser Lys
           195                 200                 205
   Ala Asn Ala Leu Gly Arg Ile Gly Thr Pro Glu Glu Val Ala Asn Ala
       210                 215                 220
   Val Leu Phe Leu Ala Ser Asp Glu Ala Ser Phe Ile Thr Gly Ala Thr
   225                 230                 235                 240
   Leu Ser Val Asp Gly Gly Gly Leu Thr Phe
                   245                 250
<210> 9
   <211> 753
   <212> DNA
   <213> geobacillus thermodenitrificans DSM 465
<400> 9
   atgaggctaa aaggaaaagc ggcgattgtc accggcggcg cgagcggcat cggccgggcg    60
   acggcgattc gctttgcgga agaaggcgcc aaagtggcgg tgagcgacat caatgaggaa   120
   ggaggggaag aaacggtccg cctgattcgg gaaaaaggag gggaggcgat ttttgtccaa   180
   acggacgtag ccgattccaa gcaagtgagc cgccttgtcc aaacggcggt tgatgccttt   240
   ggcggcctac atattctctt taacaatgcc ggcatcggcc attcggaagt gcggagcacc   300
   gacttgtctg aagaagagtg ggaccgggtc atcaacgtta atttgaaagg agtgttcctt   360
   ggcatcaaat acgcggtgcc cgtgatgaag caatgcggtg gcggggccat tgtcaacaca   420
   tcgagcctgc ttggaatcaa agggaaaaag tacgaatcgg cctacaacgc ctcgaaggcc   480
   ggggtgattt tgttgacgaa aaatgcagca ttggaatatg ggaagtttaa cattcgcgtc   540
   aatgccattg caccgggggt cattgatacg aacatcatca cgccgtggaa acaagatgag   600
   cgcaaatggc cgatcatttc gaaagcgaac gccctcggcc gcatcgggac gccagaggaa   660
   gtggcgaacg cggtgttgtt tttggcgtcc gatgaagcgt cgtttatcac cggcgcgaca   720
   ttgtcggtcg acggcggcgg gctgacgttt tag                                753
<210> 10
   <211> 753
   <212> DNA
   <213> synthetic construct
<400> 10
   atgcgcctga aagggaaagc ggcaattgtg acgggtggcg ccagcggcat cgggcgcgcg    60
   actgcgatcc gttttgcaga agagggtgcg aaagttgccg ttagcgacat taacgaggaa   120
   ggcggtgagg aaaccgttcg cctgatccgt gaaaaaggcg gtgaggcaat cttcgtgcag   180
   acggatgtgg ccgactcaaa acaggtatct cgtctggttc agaccgcggt cgacgcgttt   240
   ggtggcctgc acatcctgtt caataacgcc ggcattggcc atagcgaagt gcgtagtact   300
   gatctgagcg aggaagagtg ggatcgcgtg attaacgtga acctgaaagg tgtgtttctg   360
   ggtattaagt atgcagtccc tgttatgaaa cagtgtggcg gtggtgcgat tgtgaatacc   420
   tctagtctgt tgggaatcaa agggaaaaag tatgaatcgg cctacaacgc atcgaaagcc   480
   ggcgtcatcc tgctgaccaa aaatgcggcc ctggagtatg gcaagttcaa tattcgtgtc   540
   aacgcgatcg ctccaggcgt tatcgatacc aacatcatta caccgtggaa gcaagatgaa   600
   cgcaagtggc cgattatctc caaagctaat gcgctgggcc gtatcggtac gccggaagaa   660
   gtggctaatg cggttctgtt tctggcaagc gacgaagcga gctttattac gggtgcaacc   720
   ctctccgtag atgggggcgg gttaaccttc taa                                753
<210> 11
   <211> 252
   <212> PRT
   <213> Chloroflexus aurantiacus DSM 635
<400> 11
   Met Glu Pro Pro Phe Ile Gly Lys Val Ala Leu Val Thr Gly Ala Ala
   1               5                   10                  15
   Ala Gly Ile Gly Arg Ala Ser Ala Leu Ala Phe Ala Arg Glu Gly Ala
               20                  25                  30
   Lys Val Val Val Ala Asp Val Asn Val Glu Gly Gly Glu Glu Thr Ile
           35                  40                  45
   Ala Leu Cys Arg Ala Leu Asn Thr Asp Ala Met Phe Val Arg Cys Asp
       50                  55                  60
   Val Ser Gln Arg Asp Glu Val Glu Arg Leu Ile Ala Leu Ala Val Asp
   65                  70                  75                  80
   Thr Phe Gly Arg Ile Asp Phe Ala His Asn Asn Ala Gly Ile Glu Gly
                   85                  90                  95
   Val Gln Ala Met Leu Ala Asp Tyr Pro Glu Glu Val Trp Asp Arg Val
               100                 105                 110
   Ile Glu Ile Asn Leu Lys Gly Val Trp Leu Cys Met Lys Tyr Glu Ile
           115                 120                 125
   Arg His Met Leu Lys Gln Gly Gly Gly Ala Ile Val Asn Thr Ser Ser
       130                 135                 140
   Val Ala Gly Leu Ala Gly Ser Arg Gly Val Ser Ala Tyr Val Ala Ser
   145                 150                 155                 160
   Lys His Gly Ile Val Gly Ile Thr Lys Ala Ala Ala Leu Glu Tyr Ala
                   165                 170                 175
   Arg Asn Gly Ile Arg Val Asn Ala Ile Cys Pro Gly Thr Ile His Thr
               180                 185                 190
   Ala Met Ile Asp Arg Phe Thr Gln Gly Asp Pro Gln Leu Leu Ala Gln
           195                 200                 205
   Phe Ala Glu Gly Glu Pro Ile Gly Arg Leu Gly Ser Pro Glu Glu Val
       210                 215                 220
   Ala Asn Ala Val Ile Trp Leu Cys Ser Asp Lys Ala Ser Phe Val Thr
   225                 230                 235                 240
   Gly Ala Thr Leu Ala Val Asp Gly Gly Arg Leu Ala
                   245                 250
<210> 12
   <211> 759
   <212> DNA
   <213> Chloroflexus aurantiacus DSM 635
<400> 12
   atggagccac ctttcattgg gaaggttgcg ctggtcaccg gcgcagcagc cggtattggt     60
   cgtgcttcag cactggcgtt tgcccgtgag ggtgccaagg ttgtcgttgc tgatgtgaat    120
   gtcgagggcg gggaagagac gattgcgctg tgtcgggctt tgaataccga tgcaatgttc    180
   gtgcgttgtg atgtttcgca acgcgatgaa gtggagcgat taattgctct ggcagttgac    240
   acgttcggtc ggatcgactt tgcgcacaac aacgccggga ttgaaggcgt gcaggcaatg    300
   ctggccgatt atcccgaaga ggtctgggat cgggtgatcg agatcaacct caaaggggtc    360
   tggttgtgta tgaagtacga aatccggcac atgctcaagc agggtggcgg tgcgattgtg    420
   aatacctcat cggtcgccgg tctggccgga tcacgtggcg tttcggcgta tgtagccagc    480
   aagcacggta ttgttggtat taccaaagcg gcagcccttg agtatgcgcg taacggtatt    540
   cgtgtcaacg caatctgtcc aggtacgatt catactgcga tgatcgaccg ctttacccag    600
   ggtgatcccc aactgcttgc ccagttcgct gagggtgaac cgattggtcg gctcggctcg    660
   cctgaagagg tcgccaatgc ggtgatctgg ctctgctcag ataaggcttc gtttgtgacc    720
   ggagcgacac tggcggttga tggtggccgc ctggcgtaa                           759
<210> 13
   <211> 759
   <212> DNA
   <213> synthetic construct
<400> 13
   atggagcccc catttatcgg gaaagttgcg ttagttacgg gggcagcggc agggatcggc      60
   agggcgagtg ccctggcgtt tgctagagaa ggggccaagg tcgttgtggc agacgttaac     120
   gtagagggtg gcgaagagac aattgcttta tgcagagctc tcaacactga tgccatgttc     180
   gtccgctgtg atgtgtcaca gcgagacgaa gtcgaaaggc taatcgccct agcggtagac     240
   acattcggcc gtattgactt tgctcataat aacgcgggca tagagggagt acaagcaatg     300
   ttggctgact atcctgagga agtatgggat cgagtaattg aaatcaatct caaggggttt    360
   tggctgtgta tgaagtacga aataaggcac atgctcaagc aaggtggcgg agcgatcgta     420
   aacactagct ctgtcgccgg tctagcagga tctcgggggg tttccgcata cgtcgcctcg     480
   aaacacggca ttgtagggat taccaaagct gcagcccttg agtatgcccg aaatggaata     540
   agagtgaatg ctatctgccc aggcacaata catactgcaa tgatagatcg gtttacgcag     600
   ggtgatccgc aacttttggc gcagttcgcc gaaggtgagc ctataggtcg ccttggtagc     660
   ccggaagagg tcgctaatgc ggtgatttgg ttgtgttcag acaaagcaag tttcgtgacg     720
   ggagctaccc tggcagtgga tggaggacgt ttagcttaa                            759
<210> 14
   <211> 243
   <212> PRT
   <213> Candida magnoliae CBS 6396
<400> 14
   Met Ser Ala Thr Ser Asn Ala Leu Ile Thr Gly Ala Ser Arg Gly Met
   1               5                   10                  15
   Gly Glu Ala Thr Ala Ile Lys Leu Ala Leu Glu Gly Tyr Ser Val Thr
               20                  25                  30
   Leu Ala Ser Arg Gly Ile Glu Gln Leu Asn Ala Ile Lys Glu Lys Leu
           35                  40                      45
   Pro Ile Val Lys Lys Gly Gln Gln His Tyr Val Trp Gln Leu Asp Leu
       50                  55                  60
   Ser Asp Ile Glu Ala Ala Ser Thr Phe Lys Gly Ala Pro Leu Pro Ala
   65                  70                  75                      80
   Ser Ser Tyr Asp Val Phe Phe Ser Asn Ala Gly Val Val Asp Phe Ala
                   85                  90                      95
   Pro Phe Ala Asp Gln Ser Glu Thr Ala Gln Lys Asp Leu Phe Thr Val
               100                 105                 110
   Asn Leu Leu Ser Pro Val Ala Leu Thr Lys Thr Ile Val Lys Ala Ile
           115                 120                 125
   Ala Asp Lys Pro Arg Glu Thr Pro Ala His Ile Ile Phe Thr Ser Ser
       130                 135                 140
   Ile Val Gly Ile Arg Gly Val Pro Asn Val Ala Val Tyr Ser Ala Thr
   145                 150                 155                     160
   Lys Gly Ala Ile Asp Ser Phe Ala Arg Ser Leu Ala Arg Glu Phe Gly
                   165                 170                     175
   Pro Lys Asn Ile His Val Asn Cys Val Asn Pro Gly Thr Thr Arg Thr
               180                 185                 190
   Glu Met Thr Lys Gly Val Asp Leu Ala Ala Phe Gly Asp Val Pro Ile
           195                 200                 205
   Lys Gly Trp Ile Glu Val Asp Ala Ile Ala Asp Ala Val Leu Phe Leu
       210                 215                 220
   Ile Lys Ser Lys Asn Ile Thr Gly Gln Ser Leu Val Val Asp Asn Gly
   225                 230                 235                     240
   Phe Gly Val
<210> 15
   <211> 732
   <212> DNA
   <213> Candida magnoliae CBS 6396
<400> 15
   atgtctgcta cttcgaacgc tcttatcact ggtgccagcc gcggaatggg cgaggccaca      60
   gctattaagc ttgcccttga ggggtacagc gtcacccttg catcacgcgg tattgagcag     120
   ctcaatgcca tcaaggaaaa actacccatc gtgaagaagg gccagcagca ctacgtttgg   180
   cagctcgatc ttagtgacat cgaggcggct tccaccttca agggggctcc tctgcctgcc   240
   agcagctacg acgtgttctt cagcaacgcc ggtgtggtgg actttgctcc gttcgcagac   300
   caaagcgaga ctgcgcaaaa ggacctgttc acggttaacc tgctgtcgcc tgttgcgttg   360
   accaagacca ttgttaaggc catcgccgac aagccccgcg agacgcctgc tcacattatc   420
   ttcacctcgt ccattgtcgg aattcgcggt gttcccaacg tggcggtcta cagcgccacc   480
   aagggcgcga ttgacagctt tgcgcgctcg cttgctcgtg agttcggtcc caagaacatc   540
   cacgttaact gcgtgaaccc gggcacgacg cgcaccgaga tgacaaaggg cgttgatctc   600
   gcggctttcg gcgatgttcc tatcaagggc tggatcgagg tcgatgcgat tgccgacgct   660
   gtgctgtttt tgatcaagtc caagaacatc actggccagt cgctcgttgt tgacaacgga   720
   ttcggtgttt aa                                                       732